# EUROPÄISCHE PATENTSCHRIFT

(11) **EP 1 079 755 B1**
(45) Veröffentlichungstag und Bekanntmachung des Hinweises auf die Patenterteilung: **25.02.2004**
(21) Anmeldenummer: 99958066.5
(22) Anmeldetag: 18.11.1999
(51) Int. Cl.: A61B 18/14

(54) **CHIRURGISCHE BIPOLARSCHERE**
SURGICAL BIPOLAR SCISSORS
CISEAU BIPOLAIRE CHIRURGICAL

(30) Priorität: 03.12.1998 DE 19855812
(43) Veröffentlichungstag der Anmeldung: 07.03.2001
(73) Patentinhaber: Aesculap AG & Co. KG, 78532 Tuttlingen (DE)
(72) Erfinder: MAYENBERGER, Rupert, D-78239 Rielasingen (DE)
(74) Vertreter: Böhme, Ulrich, Dr. Dipl.-Phys.
(86) Internationale Anmeldenummer: PCT/EP1999/008857
(87) Internationale Veröffentlichungsnummer: WO 2000/032128

(56) Entgegenhaltungen:
- EP-A- 0 850 598
- US-A- 5 573 534
- US-A- 5 954 720

## Beschreibung

Die Erfindung betrifft eine chirurgische Bipolarschere mit zwei gegeneinander verschwenkbaren Scherblättern, von denen das erste aus einem elektrisch leitfähigen Material besteht und das zweite auf seiner dem ersten Scherblatt zugewandten Seiten aus einem elektrisch isolierenden Material, auf der dem ersten Scherblatt abgewandten Seite dagegen aus einem elektrisch leitenden Material.

Chirurgische Bipolarscheren dieser Art sind beispielsweise aus der US 5,324,289 bekannt. Es gelingt mit Bipolarscheren dieser Bauart, hämostatische Schnitte durchzuführen, d.h. beim Anlegen einer Wechselspannung an die beiden elektrisch leitenden Teile der beiden Scherblätter fließt im Bereich der Schnittstelle zwischen diesen ein Strom, der durch das Vorsehen einer isolierenden Schicht auf dem einen Scherblatt nicht direkt von Scherblatt zu Scherblatt verläuft, sondern durch das im Schnittstellenbereich angeordnete Gewebe, in dem auf diese Weise Blutungen gestillt werden können.

Bei den bekannten chirurgischen Bipolarscheren werden auf eines der beiden Scherblätter im Schneidbereich isolierende Materialien aufgelegt, beispielsweise durch eine Beschichtung in einem Gasstrom oder durch Auflegen dünner Keramikschichten, die mit dem metallischen Scherblatt verbunden werden.

Ausgehend von diesem Stand der Technik liegt der Erfindung die Aufgabe zugrunde, eine chirurgische Bipolarschere im Schneidenbereich einerseits besonders wirksam und andererseits besonders stabil auszubilden.

Diese Aufgabe wird bei einer chirurgischen Bipolarschere der eingangs beschriebenen Art erfindungsgemäß dadurch gelöst, daß das zweite Scherblatt einen keramischen Formkörper in Form eines vollständigen Scherblattes umfaßt, der sich über eine Lagerstelle der beiden Scherblätter hinweg erstreckt, daß sich an die Außenseite des Formkörpers ein metallisches Stützblatt flächig anlegt, welches sich ebenfalls über die Lagerstelle hinweg erstreckt, und daß der Formkörper und das Stützblatt an ihrer Anlagefläche dauerhaft miteinander verbunden sind.

Im Gegensatz zu bekannten Bipolarscheren wird also das zweite Scherblatt selbst als keramischer Formkörper ausgebildet, so daß im Werkzeugbereich ein metallisches Scherblatt einem Scherblatt aus einem keramischen Werkstoff gegenüberliegt, diese beiden Teile führen zusammen den Schneidvorgang aus.

Zur Stabilisierung des keramischen Formkörpers und zur Ausbildung einer Gegenelektrode für das metallische Scherblatt wird ein zusätzliches Stützblatt vorgesehen, welches sich an die Außenseite des keramischen Formkörpers anlegt und mit diesem flächig verbunden ist. Dabei erstrecken sich sowohl der keramische Formkörper als auch das außenseitige Stützblatt über die Lagerstelle des Scherblattes hinweg, so daß die stützende Wirkung des Stützblattes gerade in diesem mechanisch sehr beanspruchten Bereich zur Geltung kommt.

Bei einer bevorzugten Ausführungsform kann vorgesehen sein, daß der Formkörper eine Lagerhülse ausbildet, die eine Lagerwelle der beiden Scherblätter umgibt und sich durch das erste Scherblatt hindurch erstreckt. Dadurch wird eine vollständige Isolierung der beiden Scherblätter voneinander gewährleistet, und zwar auch im Lagerbereich. Es ist dadurch möglich, eine Lagerwelle aus leitfähigem Material, insbesondere eine metallische Lagerwelle, zu benutzen, die dann auch gleichzeitig als elektrische Verbindung des Stützblattes mit einem Anschluß einer Spannungsquelle verwendet werden kann.

Bei einer bevorzugten Ausführungsform ist vorgesehen, daß der Formkörper und das Stützblatt formschlüssig ineinandergreifende Vor- bzw. Rücksprünge aufweisen. Durch diese formschlüssige Verbindung erfolgt eine optimale Unterstützung des keramischen Formkörpers, so daß die flächige Verbindung zwischen Formkörper einerseits und Stützblatt andererseits auch bei hoher Belastung erhalten bleibt.

Insbesondere kann vorgesehen sein, daß der Formkörper eine Längsrippe trägt, die in eine Längsnut des Stützblattes formschlüssig eingreift. Vorzugsweise erstrekken sich Längsrippe und Längsnut über die gesamte Länge des Formkörpers.

Weiterhin kann vorgesehen sein, daß an dem Formkörper im Bereich von dessen Lagerstelle auf der dem Stützblatt gegenüberliegenden Seite ein Verlängerungselement aus Metall anliegt und mit diesem verbunden ist, welches seinerseits mit einem Teil eines Schwenkmechanismus zum Verschwenken der Scherblätter verbunden ist. Der Schwenkmechanismus muß große Kräfte übertragen, so daß es günstig ist, hier Metall als Material zu verwenden, dieses Metall ist jedoch durch den zwischenliegenden Formkörper vom Stützblatt elektrisch isoliert, so daß sichergestellt ist, daß zwischen dem Schwenkmechanismus einerseits und dem Stützblatt andererseits keine elektrische Verbindung auftritt.

Günstig ist es dabei, wenn Formkörper und Verlängerungselement formschlüssig ineinandergreifende Vor- bzw. Rücksprünge aufweisen, so daß auch dadurch die Verbindung zwischen den beiden Teilen stabilisiert wird, insbesondere zur Übertragung der Schwenkmomente.

Der Formkörper und das Stützblatt können in beliebiger Weise flächig miteinander verbunden sein, insbesondere sind sie miteinander verlötet.

Dabei ist es günstig, wenn der Formkörper und das Stützblatt im Schneidbereich durch eine Weichlötung miteinander verbunden sind, im Bereich der Lagerstelle durch eine Hartlötung.

Insgesamt erhält man also ein sandwichartiges zweites Scherblatt, welches aus dem Formkörper und Stützblatt aufgebaut ist und welches ebenso wie das erste Scherblatt als Baueinheit in das chirurgische Instrument eingesetzt werden kann.

Die nachfolgende Beschreibung bevorzugter Ausführungsformen dient im Zusammenhang mit der Zeichnung der näheren Erläuterung. Es zeigen:

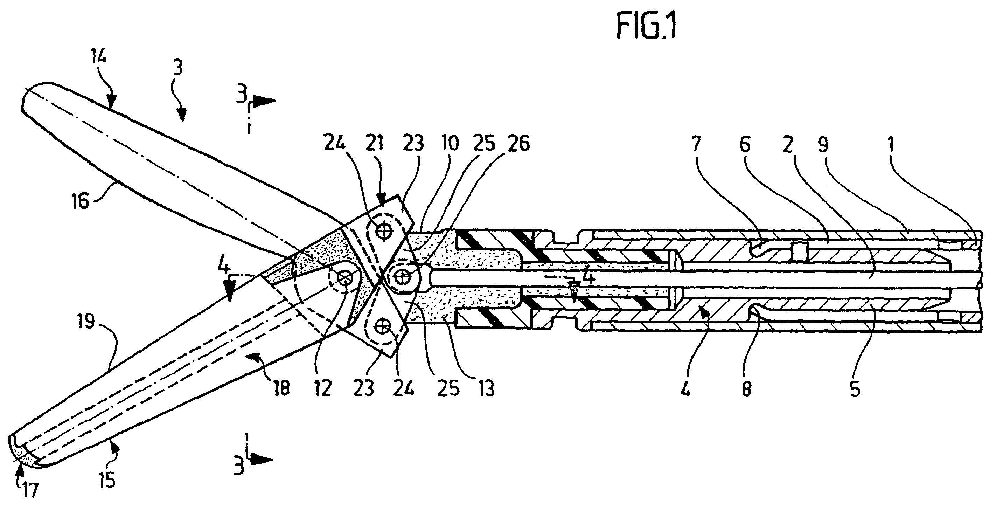

- Figur 1:: eine Längsschnittansicht durch das werkzeugseitige Ende eines bipolaren Schneidelementes mit geöffneten Scherblättern;

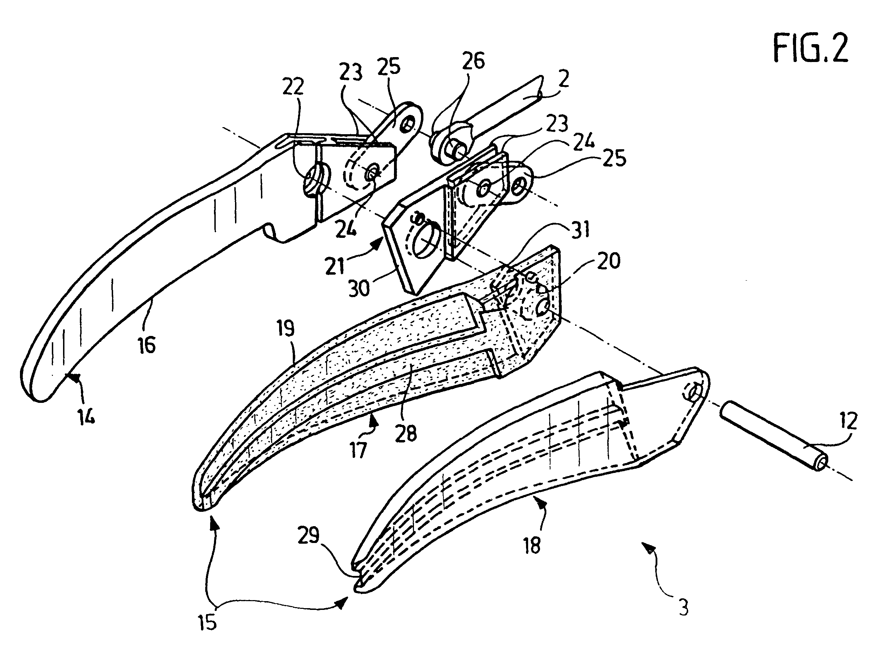

- Figur 2:: eine Explosionsansicht der das Schneidwerkzeug bildenden Teile;
- Figur 3:: eine Schnittansicht längs Linie 3-3 in Figur 1 mit zwischen den Scherblättern angeordnetem Gewebe und

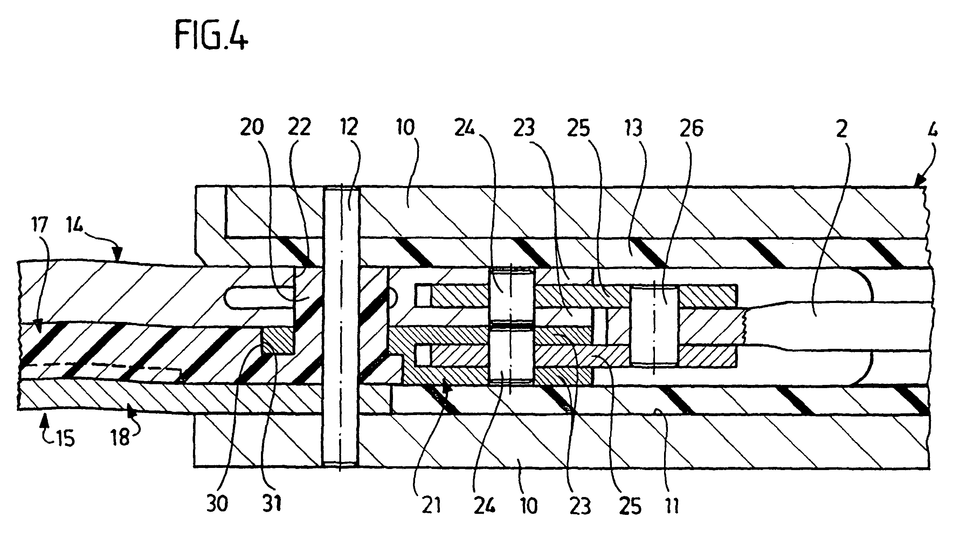

- Figur 4:: eine Schnittansicht längs Linie 4-4 in Figur 1.

Die in der Zeichnung dargestellte Bipolarschere ist als Rohrschaftinstrument ausgebildet und in der Zeichnung nur im vorderen, d.h. im Schneidbereich dargestellt. Derartige Rohrschaftinstrumente weisen einen länglichen, rohrförmigen Schaft 1 auf, in dem eine Schub- und Zugstange 2 reziprozierend verschiebbar ist. Am hinteren, in der Zeichnung nicht dargestellten Ende befindet sich ein Griffteil mit zwei gegeneinander bewegbaren Griffen, die bei entsprechender Bewegung die Schub- und Zugstange im Schaft hin- bzw. herschieben.

Am vorderen Ende des Schaftes 1, also an dessen dem Handgriff gegenüberliegenden Ende, ist ein Werkzeug 3 angeordnet, welches an einem Halter 4 gelagert ist, der mittels eines hohlen Einschubzapfens 5 stirnseitig in den Schaft 1 eingesteckt ist. Im eingeschobenen Zustand wird der Einschubzapfen 5 durch federzungenförmige Abschnitte 6 des Schaftes 1 festgelegt, die mit ihren freien Enden 7 in eine Ringnut 8 des Einschubzapfens 5 eintauchen und durch eine den Schaft 1 umgreifende Hülse 9 in dieser Lage fixiert werden.

Der Halter 4 bildet in seinem aus dem Schaft 1 hervorstehenden Teil zwei parallele Arme 10 aus, die an ihrem freien Ende durch eine den Zwischenraum 11 zwischen den Armen 10 überbrückende Lagerwelle 12 miteinander verbunden sind. Der Halter 4 und die Lagerwelle 12 bestehen aus Metall, sind also elektrisch leitend. Innenseitig sind die Arme 10 mit einer elektrisch isolierenden Beschichtung 13 versehen, die sich auch im hohlen Inneren des Einschubzapfens 5 fortsetzt.

Das Werkzeug 3 umfaßt zwei Scherblätter 14 und 15, die mittels der Lagerwelle 12 gegeneinander verschwenkbar am Halter 4 gelagert sind.

Dabei besteht das erste Scherblatt 14 aus Metall, beispielsweise aus rostfreiem Stahl, es ist geringfügig gebogen und bildet an seiner Unterseite eine Schneidkante 16 aus.

Das zweite Scherblatt 15 ist als Verbundkörper ausgebildet und umfaßt einen scherblattförmigen Formkörper 17 aus Keramik, insbesondere einer Spritzkeramik, und ein formmäßig an den Formkörper 17 angepaßtes metallisches Stützblatt 18, welches außenseitig flächig an dem Formkörper 17 anliegt. Der Formkörper 17 bildet das eigentliche Schneidelement aus, die Oberkante des Formkörpers 17 bildet eine Schneidkante 19, die mit der Schneidkante 16 des ersten Scherblattes 14 zusammenwirkt. Diese Schneidkante 19 ist dabei normalerweise stumpf ausgebildet, beispielsweise unter Verwendung eines Schneidwinkels zwischen 80 und 90°.

Der Formkörper 17 trägt an seinem rückwärtigen Ende eine aus seiner Ebene senkrecht abstehende, angeformte Hülse 20, welche die Lagerwelle 12 umgibt und somit eine Schwenklagerung für den Formkörper 17 ausbildet. Diese Hülse 20 ragt durch ein plattenförmiges Verlängerungselement 21 hindurch, welches im Bereich der Lagerstelle die Hülse 20 umgebend flächig an dem Formkörper 17 anliegt, und zwar auf der dem Stützblatt 18 abgewandten Seite des Formkörpers 17, so daß der Formkörper 17 im Lagerbereich zwischen dem metallischen Stützblatt 18 einerseits und dem ebenfalls metallisch ausgebildeten Verlängerungselement 21 eingebettet ist.

Weiterhin ragt die Hülse 20 durch eine Lageröffnung 22 im hinteren Ende des ersten Scherblattes 14 hindurch, das dadurch auf der Hülse 20 und damit konzentrisch zur Lagerwelle 12 schwenkbar am Halter 4 gelagert wird.

Sowohl das Verlängerungselement 21 als auch das erste Scherblatt 14 enden auf ihrer dem Schaft 1 zugewandten Seite in zwei parallel zueinander verlaufenden Armen 23, die jeweils zwischen sich eine Lagerwelle 24 aufnehmen. Auf dieser Lagerwelle ist jeweils ein in den Zwischenraum zwischen den Armen 23 eintauchender Hebel 25 schwenkbar gelagert, deren andere Enden schwenkbar mit Lagerstutzen 26 am freien Ende der Schub- und Zugstange 2 verbunden sind. Dadurch werden die beiden Scherblätter 14 und 15 beim Vorschieben bzw. Zurückziehen der Schub- und Zugstange 2 zwischen der Offenstellung und der Schließstellung verschwenkt, d.h. durch Verschiebung der Schub- und Zugstange 2 können die Schneidkanten 16 und 19 einander angenähert bzw. voneinander entfernt werden.

Die Schub- und Zugstange 2 besteht aus Metall und ist mit einem Pol einer Spannungsquelle verbunden, sie durchsetzt den Halter 4 durch dessen innenseitige isolierende Beschichtung 13 isoliert und verbindet die Spannungsquelle über einen der Hebel 25 und die entsprechenden Arme 23 mit dem ersten Scherblatt 14.

Zum zweiten Scherblatt 15 und insbesondere zum metallischen Stützblatt 18 besteht jedoch keine elektrische Verbindung, da das metallische Verlängerungselement 21, das in elektrisch leitender Verbindung mit der Schub- und Zugstange 2 steht, durch den keramischen Formkörper 17 und dessen angeformte Hülse 20 sowohl gegenüber dem Stützblatt 18 als auch gegenüber der Lagerwelle 12 elektrisch getrennt ist.

Andererseits trennt die Hülse 20 auch die Lagerwelle 24 von dem ersten Scherblatt 14, zwischen dem Stützblatt 18 und der Lagerwelle 12 jedoch besteht eine elektrische Verbindung, ebenso zwischen der Lagerwelle 12 und dem Halter 4, der seinerseits elektrisch leitend mit dem Schaft 1 verbunden ist. Der Schaft 1 steht mit dem zweiten Pol der Spannungsquelle in Verbindung, so daß auf diese Weise eine elektrische Verbindung von der Spannungsquelle zu dem Stützblatt 18 hergestellt wird.

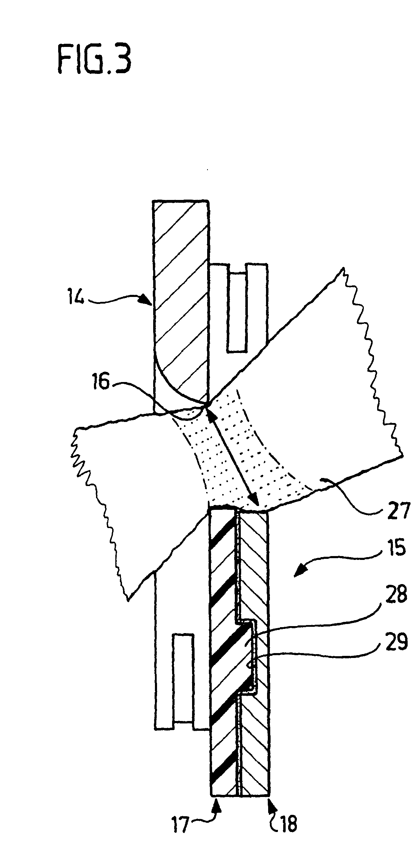

Man erhält damit zwei Elektroden in Form des ersten Scherblattes 14 und in Form des Stützblattes 18 des zweiten Scherblattes 15, die durch den Formkörper 17 elektrisch voneinander getrennt sind, die sich jedoch beim Schneiden eines zwischen den Scherblättern angeordneten Gewebes 27 so nahe kommen, daß durch das Gewebe hindurch ein Strom fließen kann, der das Gewebe hämostatiert (Figur 3).

Der keramische Formkörper 17 ist mit dem Stützblatt 18, an dem er flächig anliegt, dauerhaft verbunden, beispielsweise durch eine Verklebung oder insbesondere durch eine Verlötung. Dabei erfolgt die Verlötung im Bereich außerhalb der Lagerstelle vorzugsweise als Weichlötung, im Bereich der Lagerstelle dagegen als Hartlötung.

Die Festigkeit der Verbindung zwischen Formkörper 17 und Stützblatt 18 wird weiterhin dadurch erhöht, daß der Formkörper 17 eine sich über dessen gesamte Länge erstreckende Längsrippe an seiner Außenseite trägt, die in eine komplementäre Längsnut im Stützblatt 18 eintaucht. Auf diese Weise ergibt sich ein Formschluß zwischen Formkörper 17 und Stützblatt 18, so daß auch große Drehmomente über diesen Verbundkörper gefahrlos übertragen werden können.

Das Verlängerungselement 21 liegt mit einer Stirnkante 30 an einer entsprechenden Anschlagkante 31 des Formkörpers 17 an, so daß zusätzlich zu der Lötverbindung auch über diese Anlageflächen Drehmomente übertragen werden können, auch dadurch wird die Festigkeit des gesamten Scherblattes erhöht.

## Patentansprüche

1. Chirurgische Bipolarschere mit zwei gegeneinander verschwenkbaren Scherblättern (14, 15), von denen das erste (14) aus einem elektrisch leitfähigen Material besteht und das zweite (15) auf seiner dem ersten Scherblatt (14) zugewandten Seiten aus einem elektrisch isolierenden Material, auf der dem ersten Scherblatt (14) abgewandten Seite dagegen aus einem elektrisch leitenden Material, **dadurch gekennzeichnet, daß** das zweite Scherblatt (15) einen keramischen Formkörper (17) in Form eines vollständigen Scherblattes umfaßt, der sich über eine Lagerstelle (12) der beiden Scherblätter (14, 15) hinweg erstreckt, daß sich an die Außenseite des Formkörpers (17) ein metallisches Stützblatt (18) flächig anlegt, welches sich ebenfalls über die Lagerstelle (12) hinweg erstreckt, und daß der Formkörper (17) und das Stützblatt (18) an ihrer Anlagefläche dauerhaft miteinander verbunden sind.

2. Bipolarschere nach Anspruch 1, **dadurch gekennzeichnet, daß** der Formkörper (17) eine Lagerhülse (20) ausbildet, die eine Lagerwelle (12) der beiden Scherblätter (14, 15) umgibt und sich durch das erste Scherblatt (14) hindurch erstreckt.

3. Bipolarschere nach einem der voranstehenden Ansprüche, **dadurch gekennzeichnet, daß** der Formkörper (17) und das Stützblatt (18) formschlüssig ineinandergreifende Vor- bzw. Rücksprünge (28; 29) aufweisen.

4. Bipolarschere nach Anspruch 3, **dadurch gekennzeichnet, daß** der Formkörper (17) eine Längsrippe (28) trägt, die in eine Längsnut (29) des Stützblattes (18) formschlüssig eingreift.

5. Bipolarschere nach einem der voranstehenden Ansprüche, **dadurch gekennzeichnet, daß** an dem Formkörper (17) im Bereich von dessen Lagerstelle (12) auf der dem Stützblatt (18) gegenüberliegenden Seite ein Verlängerungselement (21) aus Metall anliegt und mit diesem verbunden ist, welches seinerseits mit einem Teil (25) eines Schwenkmechanismus (25, 2) zum Verschwenken der Scherblätter (14, 15) verbunden ist.

6. Bipolarschere nach Anspruch 5, **dadurch gekennzeichnet, daß** Formkörper (17) und Verlängerungselement (21) formschlüssig ineinandergreifende Vor- bzw. Rücksprünge (30, 31) aufweisen.

7. Bipolarschere nach einem der voranstehenden Ansprüche, **dadurch gekennzeichnet, daß** der Formkörper (17) und das Stützblatt (18) miteinander verlötet sind.

8. Bipolarschere nach Anspruch 7, **dadurch gekennzeichnet, daß** der Formkörper (17) und das Stützblatt (18) im Schneidbereich durch eine Weichlötung miteinander verbunden sind, im Bereich der Lagerstelle (12) durch eine Hartlötung.

## Claims

1. Surgical bipolar scissors comprising two mutually pivotable scissor blades (14, 15), of which the first (14) is made of an electrically conductive material and the second (15) is made of an electrically insulating material on its side facing the first scissor blade (14), but is made of an electrically conductive material on the side remote from the first scissor blade (14), **characterised in that** the second scissor blade (15) comprises a ceramic shaped part (17) in the form of a complete scissor blade extending beyond a pivot point (12) of the two scissor blades (14, 15), **in that** a metallic supporting blade (18) lies flat against the outside of the shaped part (17) and likewise extends beyond the pivot point (12), and **in that** the shaped part (17) and the supporting blade (18) are permanently connected to one another over their contact surfaces.

2. Bipolar scissors according to claim 1, **characterised in that** the shaped part (17) forms a bearing sleeve (20) which surrounds a bearing shaft (12) of the two scissor blades (14, 15) and extends through the first scissor blade (14).

3. Bipolar scissors according to any one of the preceding claims, **characterised in that** the shaped part (17) and the supporting blade (18) have positively mutually engaging projections and recesses (28; 29).

4. Bipolar scissors according to claim 3, **characterised in that** the shaped part (17) carries a longitudinal rib (28) positively engaging in a longitudinal groove (29) of the supporting blade (18).

5. Bipolar scissors according to any one of the preceding claims, **characterised in that** an extension piece (21) made of metal lies against the shaped part (17) in the region of its pivot point (12) on the opposite side from the supporting blade (18) and is connected to the shaped part (17), the extension piece (21) itself being connected to a part (25) of a pivoting mechanism (25, 2) for pivoting the scissor blades (14, 15).

6. Bipolar scissors according to claim 5, **characterised in that** the shaped part (17) and the extension piece (21) have positively mutually engaging projections and recesses (30, 31).

7. Bipolar scissors according to any one of the preceding claims, **characterised in that** the shaped part (17) and the supporting blade (18) are soldered together.

8. Bipolar scissors according to claim 7, **characterised in that** the shaped part (17) and the supporting blade (18) are joined together by soft soldering in the cutting region and by hard soldering in the region of the pivot point (12).

## Revendications

1. Ciseaux bipolaires chirurgicaux comportant deux lames (14, 15) pouvant pivoter l'une par rapport à l'autre et dont la première (14) est réalisée en un matériau électriquement conducteur et la seconde (15) est réalisée en un matériau électriquement isolant sur son côté tourné vers la première lame (14) et est réalisée par un matériau électriquement conducteur sur le côté opposé à la première lame, **caractérisés en ce que** la seconde lame (15) des ciseaux comprend un corps céramique moulé (17) se présentant sous la forme d'une lame complète des ciseaux, qui s'étend au-delà d'un point de support (12) des deux lames (14, 15) des ciseaux, que sur le côté extérieur du corps moulé (17) est appliquée, à plat, sur une certaine surface, une lame d'appui métallique (18), qui s'étend également au-delà du point de support (12), et que le corps moulé (17) et la lame d'appui (18) sont reliées entre elles de façon permanente au niveau de leur surface d'application.

2. Ciseaux bipolaires selon la revendication 1, **caractérisés en ce que** le corps moulé (17) forme un manchon de support (20), qui entoure un arbre de support (12) des deux lames (14, 15) des ciseaux et traverse la première lame (14).

3. Ciseaux bipolaires selon l'une des revendications précédentes, **caractérisés en ce que** le corps moulé (17) et la lame de support (18) comportent des parties saillantes et des renfoncements (28; 29), qui engrènent réciproquement selon une liaison par formes complémentaires.

4. Ciseaux bipolaires selon la revendication 3, **caractérisés en ce qu** le corps moulé (17) porte une nervure longitudinale (28), qui s'engage selon une liaison par formes complémentaires dans une rainure longitudinale (29) de la lame d'appui (18).

5. Ciseaux bipolaires selon l'une des revendications précédentes, **caractérisés en ce qu'**un élément de prolongement (21) formé d'un métal s'applique sur le corps moulé (17), dans la zone de son point de support (12), sur la face située à l'opposé de la lame d'appui (18), et est relié à cet élément de prolongement, qui pour sa part est relié à une partie (25) d'un mécanisme pivotant (25, 2) servant à faire pivoter les lames (14, 15) des ciseaux.

6. Ciseaux bipolaires selon la revendication 5, **caractérisés en ce que** le corps moulé (17) et l'élément de prolongement (21) possèdent des parties saillantes et des renfoncements (30, 31), qui engrènent réciproquement selon une liaison par formes complémentaires.

7. Ciseaux bipolaires selon l'une des revendications précédentes, **caractérisés en ce que** le corps moulé (17) et la lame d'appui (18) sont réunis par brasage.

8. Ciseaux bipolaires selon la revendication 7, **caractérisés en ce que** le corps moulé (17) et la lame d'appui (18) sont reliés entre eux par un brasage tendre dans la zone de coupe et par un brasage dur dans la zone du point de support (12).
